# FASCICULE DE BREVET EUROPEEN

(11) **EP 0 643 978 B1**
(45) Date de publication et mention de la délivrance du brevet: **04.04.2001**
(21) Numéro de dépôt: 94401921.5
(22) Date de dépôt: 30.08.1994
(51) Int. Cl.: A61M 16/00, B29C 45/14

(54) **Sous-ensemble de réseau de conduits de gaz et appareil d'anesthésie comportant un tel sous-ensemble**
Untereinheit eines Netzes von Gasrohrleitungen und Anästhesie-Gerät
Network sub-assembly of gas conduits and anaesthesia apparatus

(30) Priorité: 09.09.1993 FR 9310702
(43) Date de publication de la demande: 22.03.1995
(73) Titulaire: TAEMA, F-92182 Antony Cédex (FR)
(72) Inventeur: Bloch, Nicolas, F-75014 Paris (FR); Guyomard, Yvon, F-95110 Sannois (FR); Jounenc, Michel, Résidence les Millepertuis, F-91940 Les Ulis (FR)
(74) Mandataire: Le Moenner, Gabriel

(56) Documents cités:
- EP-A- 0 266 964
- EP-A- 0 292 615
- US-A- 3 687 137

## Description

La présente invention concerne un sous-ensemble de réseau de conduits de gaz pour appareil de fourniture de gaz aux voies respiratoires d'un utilisateur, comprenant un corps définissant, avec un couvercle rapporté sur le corps, au moins deux portions de conduits de gaz.

Un sous-ensemble de ce type est décrit dans le document EP-0 266 964, où, pour assurer une meilleure étanchéité entre les portions de conduits de gaz qu'avec le contact plan sur plan de dispositifs antérieurs selon le document US-A-3 687 137, une feuille de matériau d'étanchéité de même surface que le couvercle est interposée entre celui-ci et le corps.

De tels sous-ensembles utilisables en particulier pour les appareils d'anesthésie, appréciés pour leurs facilités de nettoyage et de désinfection en désassemblant simplement le couvercle et le corps, peuvent présenter des architectures complexes selon le nombre des portions de conduits. Il a été récemment imaginé d'assurer l'étanchéité par des joints d'étanchéité individuels.

La présente invention a pour objet de proposer une structure de sous-ensemble perfectionnée avec un couvercle pré-équipé de cordons d'étanchéité réalisés de façon particulièrement simple, fiable et peu onéreuse.

Pour ce faire, selon une caractéristique de l'invention, les portions de conduits de gaz sont délimitées latéralement par des cloisons du corps coopérant avec des cordons d'étanchéité formés et montés par moulage/injection dans des logements du couvercle, au moins le fond de chaque logement étant totalement occupé par un cordon d'étanchéité continu.

La présente invention a également pour objet un appareil d'anesthésie comportant un tel sous-ensemble, typiquement monté de manière amovible sur l'appareil.

D'autres caractéristiques et avantages de la présente invention ressortiront de la description suivante de modes de réalisation, donnés à titre illustratif mais nullement limitatif, faite en relation avec les dessins annexés, sur lesquels:
- la figure 1 est une vue schématique d'un circuit fermé patient d'un appareil d'anesthésie partiellement constitué par un sous-ensemble selon l'invention;
- les figures 2 et 3 sont des vues en plan des faces internes coopérantes d'un couvercle et d'un corps d'un sous-ensemble selon l'invention; et
- la figure 4 est une vue schématique illustrant le procédé de réalisation des cordons d'étanchéité du couvercle de la figure 2.

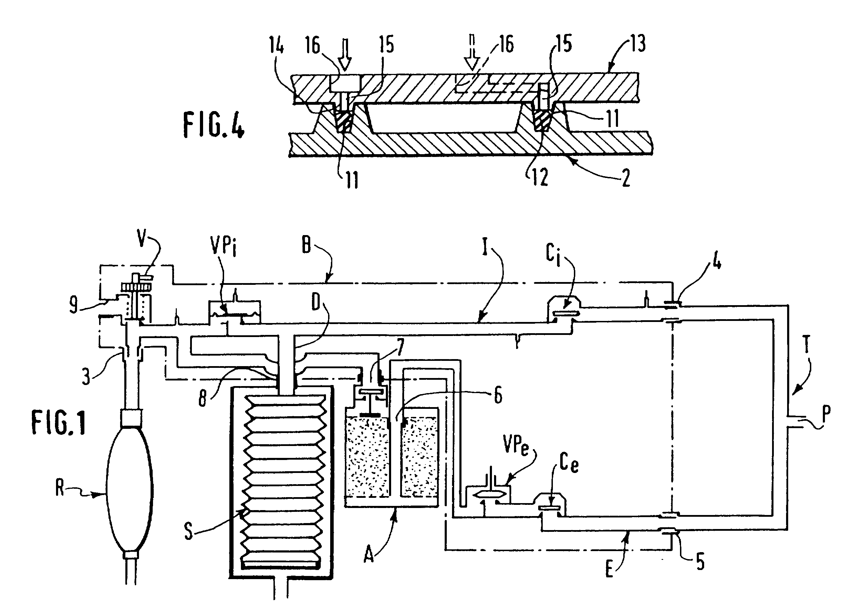

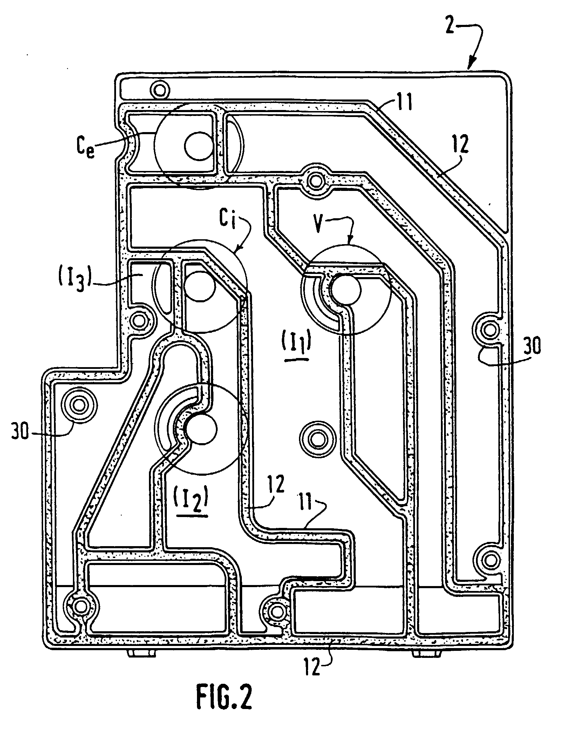

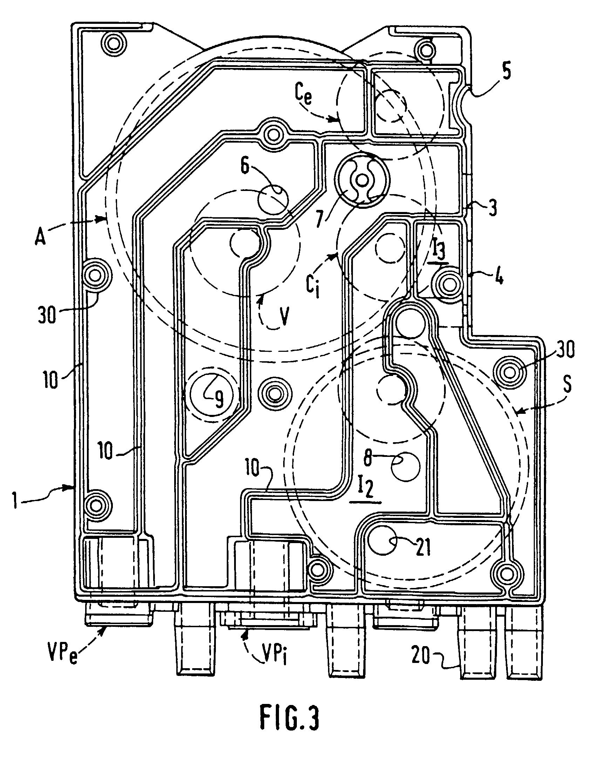

Sur la figure 1, on reconnaît les principaux éléments d'un système patient à circuit fermé d'un appareil d'anesthésie comprenant essentiellement une branche d'inspiration I, dont la partie aval est reliée à une tubulure en T, T comportant un embout P de raccordement aux voies respiratoires d'un patient, et une branche d'expiration E dont l'extrémité amont est reliée à la tubulure T et dont l'extrémité aval se raccorde à l'extrémité amont de la branche d'inspiration I dans laquelle débouche, à proximité d'une valve d'échappement tarable V, un circuit d'amenée de gaz frais oxygéné via un ballon réservoir R. La branche d'inspiration I comporte, d'amont en aval, une valve pilotée VPᵢ d'isolement du ballon R, une tubulure D de raccordement à un soufflet lesté S, et un clapet anti-retour Cᵢ. La branche d'expiration E comporte, de l'amont à l'aval, un clapet anti-retour Cₑ, une valve expiratoire pilotée VPₑ et traverse une cartouche d'adsorbant A. On a représenté sur la figure 1 les contours extérieurs du sous-ensemble B selon l'invention définissant intérieurement les portions de conduits indiquées et constituée par l'assemblage démontable d'un corps 1 de configuration sensiblement plate et caissonnée, représenté sur la figure 3, et d'un couvercle 2 sensiblement plat, représenté sur la figure 2. Comme on le voit également sur la figure 1, le sous-ensemble B, en l'occurrence le corps 1, comporte une entrée 3 de raccordement au ballon réservoir R, une sortie 4 et une entrée 5 de raccordement à la tubulure T, un passage de sortie 6 et un passage d'entrée 7 de raccordement à la cartouche d'adsorbant A, et une ouverture 8 de raccordement au soufflet S et une sortie d'échappement 9 associée à la valve V.

Sur la figure 3, on a représenté, en fantôme, les contours de la cartouche d'adsorbant A et de l'enveloppe du soufflet S qui sont rapportées sur la face inférieure du corps 1, ainsi que les contours des clapets anti-retour C_{I} et C_{E} portés par le couvercle 2. Comme on le voit sur la figure 3, le corps 1, de configuration générale parallélépipédique, comporte un certain nombre de chambres définissant des portions du système patient de la figure 1 et délimitées latéralement par des cloisons 10 à profil transversal trapézoïdal s'amincissant vers l'extérieur. On a identifié ainsi les chambres I₂ et I₃ constituant la portion de branche d'inspiration I dans le sous-ensemble B.

Comme on le voit sur la figure 2, le couvercle 2 comporte un réseau de rainures 11, également à profil trapézoïdal mais s'amincissant vers l'intérieur, correspondant exactement au réseau des nervures 10 du corps 1. Le fond des rainures 11 est totalement occupé par un cordon de joints d'étanchéité 12 injecté/moulé dans les rainures 11.

Le procédé de réalisation des joints 12 est illustré sur la figure 4 où l'on reconnaît le couvercle 2 et deux rainures 11. Sur le plan défini par les bords extérieurs des rainures 11 est appliquée une plaque de moule 13, par exemple en polyméthacrylate de méthyle, dont la face active comporte des courtes nervures 14 réparties suivant le motif des rainures 11, c'est-à-dire analogue aux nervures 10 du couvercle 1. A intervalles réguliers dans les nervures 14 débouchent des canaux d'éjection 15 communiquant, via un réseau de conduits interne à la plaque 13, avec des ouvertures 16 d'injection de matériau élastomère. Ainsi, comme représenté sur la figure 4, en positionnant la plaque moule 13 sur le couvercle 2 avec ses nervures 14 reçues dans les rainures 11, on injecte, par les orifices 16, une composition liquide de silicone, typiquement à température ambiante, qui se répartit, via les orifices d'éjection 15, dans les rainures 11 pour remplir totalement l'espace délimité, dans celle-ci, par les nervures d'injection 14. Cette opération est suivie d'une phase d'étuvage destinée à activer la polymérisation de la composition de silicone. En retirant la plaque moule 13, les cordons 12 achèvent de se solidifier et adhèrent aux rainures 11 qu'ils remplissent partiellement pour coopérer à étanchéité, en configuration de montage du bloc sous-ensemble B, avec les extrémités extérieures des nervures 10 du corps 1 lors du montage en position du couvercle 2 sur le corps 1.

Le corps 1 et le couvercle 2 sont avantageusement réalisés en matériau thermoplastique injecté/moulé, par exemple en polysulfone permettant des traitements de désinfection et de stérilisation en autoclave. La face inférieure du corps 1 est agencée pour permettre l'accrochage suspendu du boîtier de soufflet S et de la cartouche d'adsorbant A, avantageusement du type décrit dans la demande FR 2.701.400, au nom de la Demanderesse. Les ouvertures 3-5 de raccordement aux circuits gaz sont regroupées sur un côté du corps 1 et les raccordements à des organes actifs et à des circuits de capteurs sont regroupés sur une face latérale du corps 1 (au bas de la figure 3) pour permettre l'enfichage amovible du bloc sous-ensemble B dans un châssis d'appareil d'anesthésie. En particulier, comme représenté sur la figure 3, le corps 1 définit avantageusement une portion de circuit de pressurisation du soufflet S entre une entrée d'air comprimé 20 et une ouverture 21 de communication avec l'enveloppe du soufflet S. Les clapets anti-retour Cᵢ et Cₑ sont avantageusement disposés, pour les rendre visibles, sur le couvercle 2. Le couvercle 2 et le corps 1 sont typiquement assemblés par des vis reçues dans des canons tels que 30 formant également des entretoises de rigidification du bloc sous-ensemble assemblé.

Quoique l'invention ait été décrite en relation avec des modes de réalisation particuliers, elle ne s'en trouve pas limitée mais est au contraire susceptible de modifications et de variantes qui apparaîtront à l'homme de l'art.

## Revendications

1. Sous-ensemble de réseau de conduits de gaz pour appareil de fourniture de gaz aux voies respiratoires d'un utilisateur, comprenant un corps (1) définissant, avec un couvercle (2) rapporté sur le corps, au moins deux portions de conduits de gaz (I, E) délimitées latéralement par des cloisons (10) du corps, caractérisé en ce que ces cloisons coopérent avec des cordons d'étanchéité (12) formés et montés par moulage/injection dans des logements (11) du couvercle, au moins le fond de chaque logement étant totalement occupé par un cordon d'étanchéité continu.

2. Sous-ensemble selon la revendication 1, caractérisé en ce que les cordons d'étanchéité (12) sont formés en retrait dans les logements (11) et coopèrent avec des parties en saillie des cloisons (10) s'étendant partiellement dans les logements (11) en configuration assemblée du sous-ensemble.

3. Sous-ensemble selon la revendication 2, caractérisé en ce que les cloisons (10) ont une section sensiblement trapézoïdale.

4. Sous-ensemble selon l'une des revendications précédentes, caractérisée en ce que le couvercle (2) a une configuration sensiblement plate.

5. Sous-ensemble selon l'une des revendications précédentes, caractérisé en ce que le corps (1) a une configuration générale parallépipédique.

6. Sous-ensemble selon l'une des revendications précédentes, caractérisé en ce qu'au moins le corps (1) est réalisé en matériau thermoplastique moulé.

7. Sous-ensemble selon l'une des revendications précédentes, caractérisé en ce qu'il comprend des moyens (4, 5) de raccordement des portions de conduits de gaz (I, E) à au moins un circuit de gaz extérieur (R; S; T).

8. Sous-ensemble selon l'une des revendications précédentes, pour appareil d'anesthésie, caractérisé en ce qu'il définit des portions (I, E) des branches d'inspiration et d'expiration d'un circuit patient.

9. Sous-ensemble selon la revendication 8, caractérisé en ce que chaque portion de branche (I, E) comporte un clapet anti-retour (Cᵢ, Cₑ) monté dans le couvercle (2).

10. Appareil d'anesthésie comportant un sous-ensemble amovible selon l'une des revendications 1 à 9.

## Claims

1. Subassembly of a network of gas ducts for an apparatus for supplying gas to the respiratory tract of a user, comprising a body (1) which, with a lid (2) attached to the body, defines at least two portions of gas ducts (I, E) delimited laterally by partitions (10) of the body, characterized in that these partitions collaborate with sealing ribs (12) formed and mounted by moulding/injection moulding in housings (11) in the lid, at least the bottom of each housing being completely occupied by a continuous sealing rib.

2. Subassembly according to Claim 1, characterized in that the sealing ribs (12) are formed set back in the housings (11) and collaborate with projecting parts of the partitions (10) extending partially into the housings (11) when the subassembly is in the assembled configuration.

3. Subassembly according to Claim 2, characterized in that the partitions (10) have a roughly trapezoidal cross section.

4. Subassembly according to one of the preceding claims, characterized in that the cover (2) has a roughly flat configuration.

5. Subassembly according to one of the preceding claims, characterized in that the body (1) has a parallelepipedal overall configuration.

6. Subassembly according to one of the preceding claims, characterized in that at least the body (1) is made of a moulded thermoplastics material.

7. Subassembly according to one of the preceding claims, characterized in that it comprises means (4, 5) for connecting the portions of gas ducts (I, E) to at least one external gas circuit (R; S; T).

8. Subassembly according to one of the preceding claims, for an anaesthesia apparatus, characterized in that it defines portions (I, E) of the inhalation and exhalation branches of a patient circuit.

9. Subassembly according to Claim 8, characterized in that each branch portion (I, E) comprises a non-return valve (Cᵢ, Cₑ) mounted in the cover (2).

10. Anaesthesia apparatus comprising a removable subassembly according to one of Claims 1 to 9.

## Patentansprüche

1. Untereinheit eines Netzes von Gasleitungen für ein Gerät zur Zuführung von Gas zu den Atemwegen eines Benutzers, mit einem Körper (1), der mit einer an dem Körper angebrachten Abdeckung (2) mindestens zwei seitlich durch Trennwände (10) des Körpers begrenzte Gasleitungsteile (I, E) definiert, dadurch gekennzeichnet, daß diese Trennwände mit Dichtungsschnüren (12) zusammenwirken, die durch Formen/Einspritzen in Aufnahmen (11) der Abdeckung gebildet und angebracht werden, wobei zumindest der Boden jeder Aufnahme vollständig von einer durchgehenden Dichtungsschnur eingenommen wird.

2. Untereinheit nach Anspruch 1, dadurch gekennzeichnet, daß die Dichtungsschnüre (12) in den Aufnahmen (11) eingelassen ausgebildet sind und mit vorstehenden Teilen der Trennwände (10) zusammenwirken, die sich in zusammengebauter Konfiguration der Untereinheit teilweise in den Aufnahmen (11) erstrecken.

3. Untereinheit nach Anspruch 2, dadurch gekennzeichnet, daß die Trennwände (10) einen im wesentlichen trapezförmigen Querschnitt aufweisen.

4. Untereinheit nach einem der vorhergehenden Ansprüche, dadurch gekennzeichnet, daß die Abdeckung (2) eine im wesentlichen flache Konfiguration aufweist.

5. Untereinheit nach einem der vorhergehenden Ansprüche, dadurch gekennzeichnet, daß der Körper (1) eine allgemein parallelepipedische Konfiguration aufweist.

6. Untereinheit nach einem der vorhergehenden Ansprüche, dadurch gekennzeichnet, daß zumindest der Körper (1) aus geformtem thermoplastischem Material hergestellt ist.

7. Untereinheit nach einem der vorhergehenden Ansprüche, dadurch gekennzeichnet, daß sie Mittel (4, 5) zur Verbindung der Gasleitungsteile (I, E) mit mindestens einem äußeren Gaskreislauf (R; S; T) umfaßt.

8. Untereinheit nach einem der vorhergehenden Ansprüche für ein Narkosegerät, dadurch gekennzeichnet, daß sie Teile (I, E) der Inspirations- und Expirationszweige eines Patientenkreises definiert.

9. Untereinheit nach Anspruch 8, dadurch gekennzeichnet, daß jeder Zweigteil (I, E) eine in der Abdeckung (2) angebrachte Rückschlagklappe (Cᵢ, Cₑ) enthält.

10. Narkosegerät mit einer abnehmbaren Untereinheit nach einem der Ansprüche 1 bis 9.
